Europäisches Patentamt

European Patent Office

Office européen des brevets

(11) Veröffentlichungsnummer: **0 005 170**
**A2**

(12) # EUROPÄISCHE PATENTANMELDUNG

(21) Anmeldenummer: 79100980.6

(51) Int. Cl.²: **A 61 B 5/02**

(22) Anmeldetag: 02.04.79

(30) Priorität: 03.05.78 DE 2819757

(71) Anmelder: **Freiherr v. Nettelhorst, Herwig, Dipl.-Ing., PöBnecker Strasse 30, D-1000 Berlin 45 (DE)**

(43) Veröffentlichungstag der Anmeldung: 14.11.79
**Patentblatt 79/23**

(72) Erfinder: **Freiherr v. Nettelhorst, Herwig, Dipl.-Ing., PöBnecker Strasse 30, D-1000 Berlin 45 (DE)**

(74) Vertreter: **Christiansen, Henning, Dipl.-Ing., Baseler Strasse 172, D-1000 Berlin 45 (DE)**

(84) Benannte Vertragsstaaten: **BE CH DE FR GB IT NL SE**

(54) **Vorrichtung zum Erkennen von Arrhythmien.**

(57) Vorrichtung zum Erkennen von Arrhythmien im Elektrokardiogramm-Signal durch Auswertung des Zeitabstandes zwischen zeitlich aufeinanderfolgenden, für einen Herzschlag charakteristischen Signalen, mit einer Schaltung zum Erzeugen von Impulsen mit einer Frequenz, die einem Vielfachen der aktuellen Herzfrequenz mit einer Zeitkonstanten nachgeführt wird, und mindestens einer Torschaltung, die ein eine Arrhythmie anzeigendes Signal abgibt, wenn vor dem n-ten, nach dem letzten Herzschlag erzeugten Impuls ein weiteres derartiges Signal erscheint, wobei n kleiner ist als das Vielfache.

Beschreibung

Die Erfindung betrifft eine Vorrichtung zum Erkennen von Arrhythmien im Elektrokardiogramm-Signal durch Auswertung des Zeitabstandes zwischen zeitlich aufeinanderfolgenden, für einen Herzschlag charakteristischen Signalen.

Es ist bekannt, durch die zeitliche Auswertung des Erscheinens von Signalanteilen im EKG Aufschluß über die Tätigkeit des Herzens eines Patienten zu erhalten. Da die Herztätigkeit in Abhängigkeit von der Belastung, von Medikamenteneinnahmen etc. zeitabhängigen Veränderungen unterworfen ist, muß für eine genaue Diagnose das Verhalten des Herzens über einen längeren Zeitraum, der in manchen Fällen einige Tage umfassen kann, überwacht werden.

Damit nicht der Arzt oder eine Hilfsperson den Kurvenverlauf des Elektrokardiogramm fortlaufend visuell verfolgen muß, wurden automatisch arbeitende Histogrammspeicher entwickelt, welche die Zeit zwischen aufeinanderfolgenden Herzschlägen jeweils ermitteln und in Zählern nach Klassen getrennt festhalten, so daß am Ende des Überwachungszeitraums eine Übersicht über die statistische Verteilung der Herzschlagraten vorliegt.

Hierbei ist es allerdings nicht möglich, Aussagen über die Dynamik des Herzschlagverhaltens zu machen, d.h. anzugeben, wie sich die Herzrate im Laufe der Zeit verändert hat. So kann beispielsweise nicht unterschieden werden, ob bei einem Patienten jeweils über einen längeren Zeitraum innerhalb der Überwachungszeit sowohl Bradykardien als auch und Tachykardien vorlagen oder ob es sich um Doppelschlagphänomene, d.h. einen Wechsel zwischen zwei Herzschlagraten, jeweils von einem Herzschlag auf den anderen, gehandelt hat.

Es ist in diesem Zusammenhang versucht worden, durch Aufnahme sogenannter Differenz-Histogramme eine Aussage über die Änderung der Herztätigkeit von einem Herzschlag auf den anderen dadurch zu erhalten, daß die Zeiten zwischen zwei aufeinanderfolgenden Herzschlägen jeweils miteinander verglichen werden. Beim Auftreten von eine vorgegebene Differenz überschreitenden Abweichungen wird ein eine Arrhythmie anzeigendes Signal ausgegeben. Dabei werden auch die Differenzen der Zeiten zwischen zwei aufeinanderfolgenden Herzschlägen nach Zeiten klassiert in verschiedenen Zählern festgehalten, so daß am Ende eines Überwachungszeitraums die statistische Verteilung der Differenzen der Zeiten zwischen jeweils zwei aufeinanderfolgenden Herzschlägen zur Verfügung steht.

Diese Lösung hat den Nachteil, daß die ermittelten absoluten Zeitdifferenzen keine getreuen Rückschlüsse auf das Herzverhalten des Patienten zulassen. Die ermittelten und registrierten Zeitdifferenzen weisen nämlich für übereinstimmende Befunde bei geringen Herzschlagraten einen weitaus größeren Wert auf als bei hohen Herzschlagraten, so daß Fehlinterpretationen nicht ausgeschlossen werden kön-

/3

nen. Bei Registrierung über einen längeren Zeitraum mit Hilfe eines Histogrammspeichers ist eine nachträgliche Zuordnung der mittels Zählern als Ereignisse festgehaltenen Zeitdifferenzen zu den unterschiedlichen aufgetretenen Herzschlagraten nicht möglich.

Aus der US-PS 3 755 783 ist ein Analysator für biomedizinische Signale bekannt, bei dem Unregelmäßigkeiten des Herzrhythmus über eine Klassierung der für Herzschläge charakteristischen Signale bezüglich der seit dem jeweils vorherigen Schlag vergangenen Zeiten ermittelt werden. Da die bei dieser Klassierung als Maßstab dienenden Zeiträume feststehend sind, müssen diese stets kleiner gewählt sein als die kürzeste Zeitdauer zwischen zwei aufeinanderfolgenden zu erwartenden regelmäßigen Herzschlägen. Weil aber bei niedriger Herzfrequenz ein in einem zeitlichen Abstand folgender Herzschlag, der bei hoher Herzfrequenz noch regelmäßig wäre, bereits eine Arrhythmie bedeutet, sind mit dem bekannten Analysator nicht alle Unregelmäßigkeiten des Herzrhythmus feststellbar.

Der Erfindung liegt die Aufgabe zugrunde, eine Vorrichtung der obengenannten Gattung zu schaffen, bei der mit Hilfe der ermittelten Daten eine zutreffende Aussage über das dynamische Verhalten des Herzens während des Überwachungszeitraums möglich ist.

Diese Aufgabe wird erfindungsgemäß mittels der im kennzeichnenden Teil des Hauptanspruchs angegebenen Merkmale gelöst.

Der Erfindung liegt die Erkenntnis zugrunde, daß eine zuverlässige Diagnose aufgrund der zeitlichen Änderungen

/4

des Herzverhaltens nur dann möglich ist, wenn die Auswertung der Herzaktivitäten derart unter Berücksichtigung der aktuellen Herzschlagfrequenz erfolgt, daß die in Betracht kommenden Zeitdifferenzen oder Vergleichszeiten sich proportional zur Herzfrequenz verhalten.

Besonders vorteilhaft ist bei der erfindungsgemäßen Lösung, daß die Vorrichtung einfach aufgebaut und unter Verwendung handelsüblicher integrierter Bauelemente in einer tragbaren Ausführung herstellbar ist, die von dem zu untersuchenden Patienten am Körper getragen werden kann. Damit ist die Beurteilung der Herztätigkeit nicht auf die körperliche Bewegungssituation bei einer Diagnose im Untersuchungsraum des Arztes beschränkt, sondern kann unter den normalen Lebensumständen des Patienten durchgeführt werden.

Weiterhin ist bei der Erfindung günstig, daß in den meisten Fällen schon nach Untersuchungszeiträumen von nur einigen Minuten eine sichere Aussage über die Herztätigkeit des Patienten möglich ist.

Bei einer vorteilhaften Weiterbildung der Erfindung gelangt ein PLL-System zur Anwendung, welches einen besonders einfachen und betriebssicheren Aufbau der erfindungsgemäßen Vorrichtung ermöglicht. Dabei dient die oberhalb der Herzfrequenz liegende Oszillatorfrequenz des PLL-Systems als Taktsignal zur Festlegung der zeitlichen Grenzwerte, welche als Normal zur Beurteilung der Herztätigkeit dienen.

Die erfindungsgemäße Vorrichtung zur Arrhythmieerkennung läßt sich mit einem Gerät zur Ermittlung der Herzfrequenz, insbesondere zur klassierten Registrierung in Form eines Histogramms, kombinieren, wobei sich die gewonnenen Aussagen über die aufgetretenen Herzfrequenzen und die damit einhergehenden Arrhythmien vorteilhaft ergänzen. Dabei ist es besonders günstig, wenn eine Vorrichtung zur Ermittlung und Registrierung des Herzfrequenzhistogramms zur Anwendung kommt, wie sie in der deutschen Patentanmeldung P 27 36 541.9 beschrieben ist. In diesem Fall läßt sich nämlich sowohl für die Messung und Registrierung der Herzfrequenz als auch für die Arrhythmieerkennung ein einziges PLL-System verwenden, so daß eine besonders ökonomische und auch energiesparende Konzeption möglich ist, was besonders für eine tragbare Ausführung wichtig ist. Für die klassenmäßige Registrierung von Herzschlagraten und Arrhythmien lassen sich gleichartige Speicher verwenden. Die Oszillatorfrequenz des PLL-Systems wird dabei zweckmäßigerweise so gewählt, daß das Vielfache gleich 60 ist.

In diesem Zusammenhang ergibt sich der weitere Vorteil, daß die Vorrichtung zur Arrhythmieerkennung in einfacher Weise als - beispielsweise mittels einer Steckverbindung zuschaltbares - Zusatzgerät für das Gerät zur Ermittlung des Histogramms ausgebildet sein kann.

Die Registrierung der ermittelten Arrhythmien erfolgt bei einer auf eine einfache Bauweise abzielenden Grundkonzeption mit einem einzigen Zähler mit direkter Ablesemöglichkeit. Für bestimmte Anwendungsfällen sind jedoch Varianten möglich, welche durch eine entsprechende logische Verknüpfung der für die Ereignisse repräsentativen Signale und geeignete Zuordnung von Zählern auch eine Klassierung der Arrhythmien in Abhängigkeit von ihrem zeitlichen Auftreten im EKG und der augenblicklichen Herzfrequenz ermöglichen.

0005170

Durch eine entsprechende Festlegung der verwendeten Torzeiten ist dabei auch die Erkennung von sehr frühzeitig
auftretenden Extrasystolen möglich, welche in die vulnerable Phase des Herzens fallen.

Bei einer anderen hervorzuhebenden Auführungsform wird bei
der Arrhythmieerkennung und -registrierung ein zusätzliches Formkriterium der Elektrodkardiogramm-Signale herangezogen, um supraventrikuläre und ventrikuläre Extrasystolen voneinander zu unterscheiden. Als Kriterium dient
dabei eine frequenzmäßige Selektion des Energiemaximums
der auftretenden Herzsignale.

Ein bevorzugtes Ausführungsbeispiel der Erfindung ist in
der Zeichnung dargestellt und wird nachfolgend näher beschrieben. Es zeigen:

Fig. 1   ein Blockschaltbild des Ausführungsbeispiels und

Fign. 2a bis 2g   Diagramme des zeitlichen Amplitudenverlaufs einiger auftretender Signale.

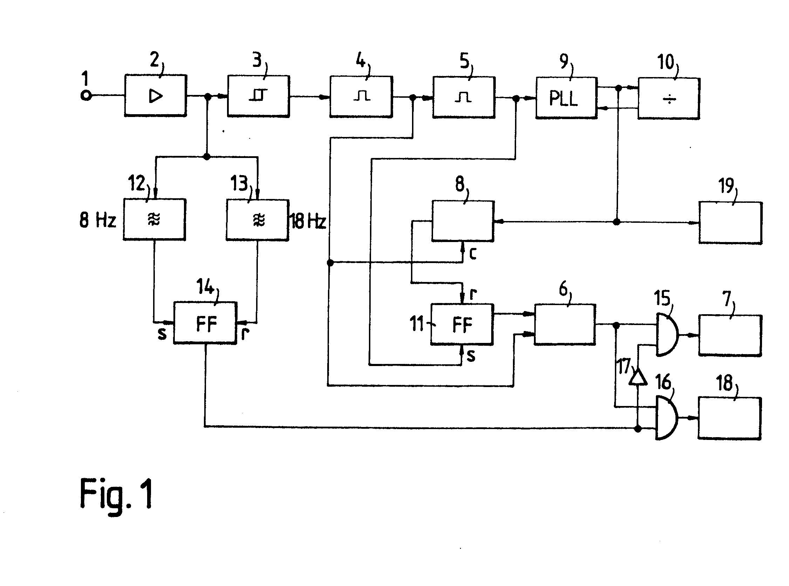

Bei dem in Fig. 1 dargestellten Blockschaltbild der erfindungsgemäßen Vorrichtung wird über einen Eingang 1 ein
über EKG-Elektroden vom Körper des Patienten abgeleitetes
Herzsignal zu einem Verstärker 2 geführt, mittels dessen
es für die nachfolgende Signalverarbeitung in seinem Pegel
heraufgesetzt wird. Das Ausgangsignal gelangt vom Verstärker 2 zu einem Schmitt-Trigger 3, an dessen Ausgang nur
solche Herzsignale als Impuls erscheinen, deren Amplitude
einen vorgegebenen Pegel überschreitet (QRS-Komplexe).

/7

Die weitere Signalverarbeitung erfolgt über einen monostabilen Multivibrator 4, der jeweils auf ein den vorgegebenen Pegel überschreitendes Herzsignal hin einen Impuls bestimmter Dauer abgibt. Dieser Impuls gelangt einerseits zu einem zweiten monostabilen Multivibrator 5 und andererseits an eine Torschaltung 6, um beim Vorliegen zusätzlicher noch zu erläuternder Bedingungen in einem Anzeigezähler 7 als Ereignis, d.h. als aufgetretene Arrhythmie, registriert zu werden.

Der monostabile Multivibrator 4 dient zur Impulsformung, wobei die Zeitdauer des Impulses so gewählt ist, daß Anteile desjenigen QRS-Komplexes, der bereits den monostabilen Multivibrator 4 ausgelöst hat, keinen erneuten, eine Arrhythmie vortäuschenden Impuls hervorrufen können.

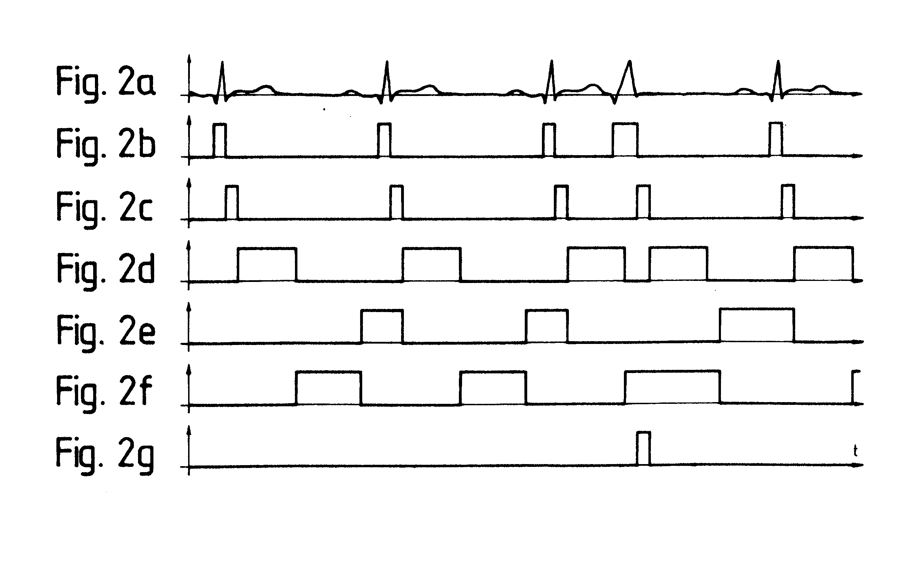

In Fig. 2a ist der Verlauf des Eingangssignals am Anschluß 1 dargestellt, während in Fig. 2b das Signal am Ausgang des Schmitt-Triggers 3 und in Fig. 2c das Signal am Ausgang des monostabilen Multivibrators 4 wiedergegeben ist.

Ein dem zweiten monostabilen Multivibrator 5 nachgeschaltetes PLL-System 9 schwingt auf einer Frequenz, die einem Vielfachen der augenblicklichen Herzfrequenz mit einer Zeitkonstante nachgeführt wird. Aufbau und Funktion eines derartigen PLL-Systems sind in der Druckschrift der Signetics Corp. "A New Phase Locked Loop With High Stability and Accuracy" von J. A. Mattis und H. R. Camenzind beschrieben. Der in dem PLL-System 9 üblicherweise enthaltene - in der Zeichnung nicht getrennt dargestellte - spannungsabhängige Oszillator wird durch das Ausgangssignal einer ebenfalls im PLL-System 9 enthaltenen Phasenvergleichsschaltung gesteuert, die einen Vergleich der Phasenlage der Ausgangssignale des monostabilen Multivibrators 5 und einer Teilerschaltung 10 durchführt. Letztere setzt dabei die Frequenz des im PLL-System 9 enthaltenen Oszillators um das Vielfache herab, so daß die von der Teilerschaltung 10 abgegebenen Impulse im wesentlichen wieder die Herzfrequenz aufweisen. (Bei dem dargestellten Ausführungsbeispiel wird das PLL-System jeweils durch die Vorderflanke der von dem monostabilen Multivibrator 5 abgegebenen Impulse synchronisiert.)

Ein die Torschaltung 6 steuerndes Flipflop 11 wird jeweils durch die Rückflanke der von dem monostabilen Multivibrator 5 abgegebenen Impulse über einen "Setz"-Eingang s gesetzt. Das Flipflop 11 beeinflußt die Torschaltung 6 in der Weise, daß die Impulse vom monostabilen Multivibrator 4 nur dann zu dem Anzeigezähler 7 gelangen, wenn es gesetzt ist, sein Ausgang sich also auf dem H-Pegel befindet. Zurückgesetzt wird das Flipflop 11 über seinen "Rücksetz"-Eingang r jeweils, wenn eine Zählschaltung 8 einen vorgegebenen Wert n erreicht hat, der kleiner ist als das Vielfache, mit dem das PLL-System 9, bezogen auf die Herzfrequenz, schwingt. Die Zählschaltung 8 kann dabei beispielsweise durch eine rücksetzbare Frequenzteilerschaltung gebildet werden, welche die vom Oszillatorausgang des PLL-Systems 9 gelangenden Impulse in ihrer Frequenz um einen Faktor herabsetzt, der gleich dem Doppelten des vorgegebenen Wertes ist, so daß, nachdem die Hälfte der für eine vollständige Periode des Ausgangssignals notwendigen Impulse am Eingang erschienen ist, der Ausgang der Zählschaltung 8 seinen logischen Zustand wechselt.

Der monostabile Multivibrator 5 dient dazu, im Elektrokardiogramm-Signal nach dem QRS-Komplex auftretende T-Wellen zu unterdrücken, so daß diese nicht zur Registrierung einer Arrhythmie führen können. Dadurch, daß das Setzen des Flipflops 11 - und damit Öffnen der Torschaltung 6 - erst mit der fallenden Flanke des von dem monostabilen Multivibrator 5 abgegebenen Impulses erfolgt, können durch T-Wellen möglicherweise hervorgerufene Signale kein unerwünschtes Heraufsetzen des Anzeigezählers 7 auslösen.

Die Arbeitsweise der erfindungsgemäßen Vorrichtung zur Arrhythmieerkennung soll nun anhand der weiteren in den Fign. 2d bis g dargestellten Impulsdiagramme erläutert werden:

Die Zählschaltung 8 definiert jeweils zwischen dem Zeitpunkt in dem sie über den "Clear"-Eingang c (Fig.1) in einen Anfangszustand gesetzt wird und dem Erreichen eines vorgegebenen Zählerstandes, getaktet durch die Teilerschaltung 10, einen Zeitraum, in dessen Verlauf Arrhythmien zuzurechnende impulsförmige Anteile des Herzsignals ein Heraufsetzen des Anzeigezählers 7 bewirken (Impulsdarstellung gemäß Fig. 2d). Diese Zeitdauer bis zum Rücksetzen der Zählschaltung 8 ist damit abhängig von der Frequenz, mit der der Oszillator des PLL-Systems 9 schwingt. Da das PLL-System mit der augenblicklichen Herzfrequenz synchronisiert ist bzw. sich bei einer Änderung derselben oder beim Auftreten von Extrasystolen dieser relativ schnell wieder anpaßt, ist der Erkennungszeitraum für mit Arrhythmien einhergehende Impulse im Herzsignal stets der augenblicklichen Herzfrequenz angepaßt.

Schwingt der Oszillator im PLL-System 9 beispielsweise mit dem Zehnfachen der Herzfrequenz, wobei das Teilerverhältnis der Teilerschaltung 10 ebenfalls zehn beträgt, so werden, wenn die Zählschaltung 8 nach sieben Impulsen ein Signal abgibt, welches das Flipflop 11 zurücksetzt, 70% der Zeit zwischen zwei QRS-Impulsen bei der augenblicklichen Herzfrequenz zur Erkennung von Arrhythmien ausgenutzt, d.h. alle anderen am Ausgang des monostabilen Multivibrators 4 einen Impuls auslösenden Herzsignale werden als regelmäßiges Herzverhalten gewertet. (Diese Zahlen können selbstverständlich entsprechend den durch die zugrundeliegende Diagnoseaufgabe gestellten Anforderungen abgewandelt werden.)

Wenn die aus der Torschaltung 6, dem Anzeigezähler 7, der Zählerschaltung 8 und dem Flipflop 11 bestehende Schaltungsgruppe mehrfach vorhanden ist und die Zählschaltungen jeweils bei unterschiedlichen Zählerständen ein Signal abgeben (d.h. Zeiträume unterschiedlicher Dauer festlegen), so läßt sich eine zeitliche Klassierung der mit den registrierten Arrhythmien einhergehenden Ereignisse im Herzsignalverlauf im Sinne eines Histogramms vornehmen. (Zweckmäßigerweise wird man in diesem Fall logische Schaltungsmittel vorsehen, welche jeweils, wenn eine der Zählschaltungen ihren vorgegebenen Zählerstand erreicht, einen anderen Anzeigezähler über die entsprechende Torschaltung zum Registrieren von Ereignissen freigeben, so daß jedes eine Arrhythmie anzeigende Signal nur in einem der Zähler festgehalten wird).

Die besonders einfache Bauweise der dargestellten Ausführungsform resultiert insbesondere aus der Tatsache, daß die Zählschaltung 8 mit Impulsen angesteuert wird, die aus dem PLL-System 9 stammen, also hinsichtlich ihrer Frequenz dem aktuellen Herzverhalten angepaßt sind, so daß keine zusätzlichen Zeitgebermittel erforderlich sind und stets Synchronität mit dem derzeitigen Herzschlagfrequenz
besteht.

Durch eine entsprechende Dimensionierung der Zeitkonstanten des PLL-Systems ist es möglich, die erfindungsgemäße
Vorrichtung so einzustellen, daß normale, langsame Veränderungen der Herzfrequenz zu keiner Anzeige führen, während Abweichungen sofort erkannt und registriert werden.
Dieser Wert ist so gewählt, daß die Frequenz der Schaltung
zum Erzeugen von Impulsen sich dem Vielfachen der aktuellen Herzfrequenz innerhalb von zwei bis drei Herzschlägen
soweit anpaßt, daß durch nachfolgende Herzschläge mit im
wesentlichen konstanter Frequenz kein eine Arrhythmie anzeigendes Signal ausgelöst wird. Die Einstellung der Zeitkonstanten erfolgt durch entsprechende Dimensionierung
eines RC-Gliedes am Steuereingang des im PLL-System 9 vorhandenen spannungsgesteuerten Oszillators (VCO).

Bei der bisherigen Beschreibung des Impulsverlaufs gemäß
Fign. 2a bis d war davon ausgegangen worden, daß der gesamte Zeitraum, in dem Ausgang der Zählschaltung 8 den logischen L-Zustand einnimmt, zur Registrierung von Arrhythmien anzeigenden Signalen zur Verfügung steht. Dieser
Zeitraum ist aber jeweils durch die Dauer der Ausgangsimpulse (Fig. 2d) des monostabilen Multivibrators 5 verkürzt, um - wie oben erwähnt - die T-Wellen zu unterdrükken. Das die Torzeiten der Torschaltung 6 festlegende
Flipflop 11 wird erst durch die Rückflanke des Ausgangsimpulses des monostabilen Multivibrators 5 wieder gesetzt
(Verlauf gemäß Fig. 2f). Durch einen dem Flipflop 11 nachgeschalteten Inverter (in der Zeichnung nicht dargestellt), ist es aber auch möglich, jeweils die gerade in
die vulnerable Phase des Herzens (bis zur T-Welle) fallenden Impulse separat auszugeben und zu registrieren, was in einigen Diagnosefällen von Bedeutung sein kann.

In Fig. 2d sind die Ausgangsimpulse des monostabilen Multivibrators 5 bei zunächst regelmäßigem Herzschlagverhalten dargestellt. Der Zählschaltung 8 wird jeweils durch die Ausgangssignale des monostabilen Multivibrators 4 (Fig. 2c) zurückgesetzt und gibt ein Ausgangssignal ab, wenn der erwähnte Zählerstand von "7" erreicht wurde. In Fig. 2f ist dargestellt, wie der Ausgang des Flipflops 11 in den Zeiten zwischen der fallenden Flanke des Ausgangssignals des monostabilen Multivibrators 5 bis zur ansteigenden Flanke des Ausgangssignals des Zählschaltung 8 den logischen H-Zustand einnimmt.

Beim Auftreten einer Extrasystole (Fig. 2a nach dem dritten QRS-Komplex) gibt der Schmitt-Trigger 3 und daraufhin auch der monostabile Multivibrator 4 einen Ausgangsimpuls ab, woraufhin die Zählschaltung 8 erneut zurückgesetzt wird. Das Flipflop 11 bleibt jedoch gesetzt, so daß über die Torschaltung 6 der Ausgangsimpuls des monostabilen Multivibrators 4 zum Anzeigezähler 7 gelangt und dort registriert wird. Der am Ausgang der Torschaltung 6 erscheinende Impuls ist in Fig. 2g dargestellt. Die verzögerte Rückflanke des Ausgangsimpulses des monostabilen Multivibrators 5 bleibt am Flipflop 11 wirkungslos, da dieses bereits gesetzt ist. Dadurch, daß die Vorderflanke des selben Impulses aber bereits das PLL-System 9 als Referenzsignal erreicht hat, erhöht sich Frequenz des darin befindlichen Oszillators, weil das System danach trachtet, sich dieser beschleunigten Herzrate anzupassen. Die Zählschaltung 8 erreicht damit schneller als bisher den vorgegebenen Wert, woraufhin das Flipflop 11 zurückgesetzt wird. Der durch den nächsten QRS-Komplex ausgelöste Impuls des monostabilen Multivibrators 4 kann die Torschaltung 6 damit nicht mehr passieren und wird vom Anzeigezähler 7 infolgedessen auch nicht erfaßt. Wenn das PLL-System der aktuellen Herzfrequenz mit einer Zeitkonstanten folgt, die so gewählt ist, daß auch bei großen Frequenzsprüngen innerhalb von zwei bis drei Herzschlägen wieder Synchronität erzielt wird, so ist einerseits gewährleistet, daß normale Frequenzänderungen des Herzens nicht als Arrhythmien gewertet werden, andererseits aber Doppelschlagphänomene sicher erkannt werden.

Durch weitere in Fig. 1 dargestellte, Schaltungsteile ist es möglich, nach Formkriterien supraventrikuläre und ventrikuläre Extrasystolen voneinander zu unterscheiden und getrennt zu registrieren. Da bei ventrikulären Extrasystolen das Energiemaximum in einem niederfrequenteren Bereich bei ca. 6 Hz und bei supraventrikulären das Energiemaximum bei einer höheren Frequenz im Bereich von ca. 18 Hz liegt, kann durch dem Verstärker 2 nachgeschaltete Filterschaltungen (Bandpaß für ca. 6 Hz 12 und Bandpaß für ca. 18 Hz 13) unterschieden werden, in welchen Frequenzbereich ein erfaßter Signalanteil fällt. Unter der Voraussetzung, daß letztere entsprechende Impulsformerschaltungen enthalten, wird durch das Ausgangssignal des Bandpasses 12 der "Setz"-Eingang s und durch das Ausgangssignal des Bandpasses 13 der "Rücksetz"-Eingang r eines Flipflops 14 angesteuert, welches mit Hilfe von UND-Gattern 15 und 16 sowie einem Inverter 17 in Abhängigkeit vom Frequenzbereich des letzten erfaßten Signals dieses entweder dem Anzeigezähler 7 oder einem weiteren Anzeigezähler 18 zuordnet. Bei der dargestellten Ausführung werden ventrikuläre Extrasystolen im Anzeigezähler 18 und supraventrikuläre Extrasystolen im Anzeigezähler 7 festgehalten.

Besonders aufschlußreich für den behandelnden Arzt ist die kombinierte Auswertung der Ergebnisse der erfindungsgemäßen Vorrichtung mit einem gleichzeitig aufgenommenen Herzfrequenzhistogramm. Zu diesem Zweck ist die Vorrichtung direkt mit einem Herzfrequenzmesser 19 eines Histogrammspeichers verbunden, wobei die Schaltungsteile für die Signalaufnahme und -verstärkung bzw. Formung nur einfach vorhanden sein müssen. Da die Herzfrequenz um das das Vielfache heraufgesetzt ist, kann hier ein üblicher digitaler Frequenzmesser mit relativ kurzer Integrationszeit verwendet werden. Aus Rationalisierungsgründen kann der Histogrammspeicher so aufgebaut sein, daß die Schaltung zur Arrhythmieerkennung nach Bedarf permanent oder auch nur zeitweise - beispielsweise mittels Steckverbindungen - hinzufügbar ist. Die gehäusemäßigen und elektrischen Verbindungsmöglichkeiten brauchen hier nicht näher erläutert zu werden, da sie dem auf dem Gebiet tätigen Fachmann geläufig sind. Dabei werden in dem Histogrammspeicher vorhandene Klassenspeicher für die Registrierung des Inhaltes des Anzeigezählers 7 bzw. 18 mitbenutzt. Bei Verwendung der erfindungsgemäßen Vorrichtung zusammen mit einem Histogrammspeicher, welches gemäß der deutschen Patentanmeldung P 27 36 541.9 arbeitet, wird das dort vorgesehene PLL-System (mit Teilerschaltung 10) mitbenutzt, wobei die Bemessung der Oszillatorfrequenz in dem PLL-System unter Berücksichtigung der Anforderungen beider Teile der zusammengefaßten Vorrichtung erfolgt. Das PLL-System benutzt dann eine Frequenz, die gleich der 60-fachen Herzfrequenz ist. Mit diesem Zahlenwert besteht die Möglichkeit, die Registrierdauer der Zählschaltung 8, d.h. die Erfassungszeiträume für die Arrythmieerkenn ng in hinreichend kleinen Stufen zu wählen. Soll die Zählschaltung 8 dagegen besonders einfach gehalten sein, so kann bei einer aus einer

/15

Teilerkette bestehenden Frequenzteilerschaltung des PLL-Systems auch eine geeignete niedrigere Frequenz abgegriffen werden.

\* \* \* \* \*

<u>P a t e n t a n s p r ü c h e</u>

1.    Vorrichtung zum Erkennen von Arrhythmien im Elektrokardiogramm-Signal durch Auswertung des Zeitabstandes zwischen zeitlich aufeinanderfolgenden, für einen Herzschlag
charakteristischen Signalen,

g e k e n n z e i c h n e t    d u r c h

eine Schaltung zum Erzeugen von Impulsen (PLL-System 9)
mit einer Frequenz, die einem Vielfachen der aktuellen
Herzfrequenz mit einer Zeitkonstante nachgeführt wird, und

mindestens eine Torschaltung (6), die ein eine Arrhythmie
anzeigendes Signal abgibt, wenn vor dem n-ten, nach dem
letzten Herzschlag erzeugten Impuls ein weiteres derartiges Signal erscheint, wobei n kleiner ist als das Vielfache.

2.    Vorrichtung nach Anspruch 1, d a d u r c h   g e -
k e n n z e i c h n e t ,  daß die Zeitkonstante beim
Nachführen derart bemessen ist, daß die Frequenz der
Schaltung zum Erzeugen von Impulsen sich dem Vielfachen
der aktuellen Herzfrequenz innerhalb von zwei bis drei
Herzschlägen soweit anpaßt, daß durch nachfolgende Herz-

schläge mit im wesentlichen konstanter .Frequenz kein eine Arrhythmie anzeigendes Signal ausgelöst wird.

3.    Vorrichtung nach einem der vorangehenden Ansprüche, d a d u r c h   g e k e n n z e i c h n e t ,   daß n zwischen dem 0,6- und dem 0,8-fachen des Vielfachen beträgt.

4.    Vorrichtung nach einem der Ansprüche 1 bis 3, d a - d u r c h   g e k e n n z e i c h n e t ,

daß als Schaltung zum Erzeugen von Impulsen mit einer Frequenz, die einem Vielfachen der aktuellen Herzfrequenz nachgeführt wird, ein PLL-System (9) vorgesehen ist, mit einem Frequenzteiler (10) zum Herabsetzen der Frequenz, die dem Vielfachen der aktuellen Herzfrequenz nachgeführt wird, um das Vielfache und

daß der Phasenvergleichsschaltung des PLL-Systems einerseits das Ausgangssignal des Frequenzteilers (10) und andererseits die aktuelle Herzfrequenz als Eingangssignale zugeführt werden.

5.    Vorrichtung nach einem der vorangehenden Ansprüche, g e k e n n z e i c h n e t   d u r c h   mindestens einen auf das eine Arrhythmie anzeigende Signal ansprechenden Zähler (7, 18).

6.    Vorrichtung nach einem der vorangehenden Ansprüche, d a d u r c h   g e k e n n z e i c h n e t ,   daß Mittel

zur logischen Verknüpfung (6, 15, 16) des eine Arrhythmie anzeigenden Signals mit mindestens einem weiteren aus dem Elektrokardiogramm-Signal abgeleiteten Signal vorgesehen sind.

7. Vorrichtung nach Anspruch 6, d a d u r c h  g e -  k e n n z e i c h n e t , daß zur Erzeugung des weiteren Signals Zeitgebermittel (5) vorgesehen sind, die, folgend auf das letzte für einen Herzschlag charakteristische Signal, für einen die nachfolgende T-Welle noch überdeckenden Zeitraum das weitere Signal abgeben.

8. Vorrichtung nach Anspruch 7, d a d u r c h  g e -  k e n n z e i c h n e t , daß Mittel zur Erzeugung eines für einen Herzschlag charakteristischen Signals einen Schmitt-Trigger (3) aufweisen und daß die Mittel zur logischen Verknüpfung (6) des eine Arrhythmie anzeigenden Signals dieses während des die nachfolgenden T-Welle noch überdeckenden Zeitraum unterdrücken.

9. Vorrichtung nach Anspruch 7, d a d u r c h  g e -  k e n n z e i c h n e t , daß Mittel zur Erzeugung eines für einen Herzschlag charakteristischen Signals einen Schmitt-Trigger (3) aufweisen und daß die Mittel zur logischen Verknüpfung (6) das während des eine die nachfolgende T-Welle noch überdeckenden Zeitraums erscheinende, eine Arrhythmie anzeigende Signal, einem gesonderten Ausgang zuführen.

/4

10. Vorrichtung nach Anspruch 6, d a d u r c h   g e -
k e n n z e i c h n e t , daß Frequenzfilter (12, 13)
vorgesehen sind, deren Ausgangssignale in Abhängigkeit von
im Herzsignal enthaltenen Frequenzanteilen das weitere
Signal auslösen, wobei durch die Mittel zur logischen Verknüpfung (15, 16) ein eine ventrikuläre Extrasystole anzeigendes Signal ausgelöst wird, wenn ein Frequenzmaximum
im Bereich von 6 Hz auftritt, und ein eine supraventrikuläre Extrassystole anzeigendes Signal, wenn ein Frequenzmaximum im Bereich von 18 Hz auftritt.

11. Vorrichtung nach einem der vorangehenden Ansprüche,
d a d u r c h   g e k e n n z e i c h n e t , daß Mittel
(19) zur Auswertung der Impulse mit einer Frequenz, die
einem Vielfachen der aktuellen Herzfrequenz mit einer
Zeitkonstante nachgeführt wird, als Maß für die augenblickliche Herzfrequenz vorgesehen sind.

12. Vorrichtung nach Anspruch 11, d a d u r c h   g e -
k e n n z e i c h n e t , daß das Vielfache gleich 60
ist und die Frequenz der Impulse pro Sekunde als Herzfrequenz pro Minute ausgegeben wird.

\* \* \* \* \*

Fig. 1

Fig. 2a

Fig. 2b

Fig. 2c

Fig. 2d

Fig. 2e

Fig. 2f

Fig. 2g

t

0005170